(19) Europäisches Patentamt
European Patent Office
Office européen des brevets

(11) EP 1 155 004 B1

(12) EUROPÄISCHE PATENTSCHRIFT

(45) Veröffentlichungstag und Bekanntmachung des Hinweises auf die Patenterteilung:
**14.01.2004 Patentblatt 2004/03**

(21) Anmeldenummer: **00916829.5**

(22) Anmeldetag: **12.02.2000**

(51) Int Cl.[7]: **C07D 261/18**
// C07C255:23

(86) Internationale Anmeldenummer:
**PCT/EP2000/001151**

(87) Internationale Veröffentlichungsnummer:
**WO 2000/050411 (31.08.2000 Gazette 2000/35)**

# (54) VERFAHREN ZUR KRISTALLISATION VON N- (4-TRIFLUORMETHYLPHENYL) -5-METHYL-ISOXAZOL- 4-CARBOXAMID

METHOD CRYSTALLISING
N-(4-TRIFLUOROMETHYLPHENYL)-5-METHYL-ISOXAZOLE-4-CARBOXAMIDE

PROCEDE DE CRISTALLISATION DE
N-(4-TRIFLUOROMETHYLPHENYL)-5-METHYL-ISOXAZOLE-4-CARBOXAMIDE

(84) Benannte Vertragsstaaten:
**AT BE CH CY DE DK ES FI FR GB GR IE IT LI LU MC NL PT SE**
Benannte Erstreckungsstaaten:
**LT LV RO SI**

(30) Priorität: **26.02.1999 DE 19908527**

(43) Veröffentlichungstag der Anmeldung:
**21.11.2001 Patentblatt 2001/47**

(73) Patentinhaber: **Aventis Pharma Deutschland GmbH**
**65929 Frankfurt am Main (DE)**

(72) Erfinder:
- **LAU, Hans-Hermann**
  **D-65812 Bad Soden (DE)**
- **HEDTMANN, Udo**
  **D-60435 Frankfurt (DE)**
- **FAASCH, Holger**
  **D-55232 Alzey (DE)**
- **GAPPISCH, Andreas**
  **D-64521 Gross-Gerau (DE)**

(56) Entgegenhaltungen:
**EP-A- 0 013 376** **DE-A- 19 734 438**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 155 004 B1

Printed by Jouve, 75001 PARIS (FR)

## Beschreibung

Die Erfindung betrifft ein Verfahren zur Gewinnung von N-(4-Trifluormethylphenyl)-5-methyl-isoxazole-4-carboxamid in kristalliner Form, das im wesentlichen frei ist von Nebenprodukten.

Die Verbindung der Formel 1

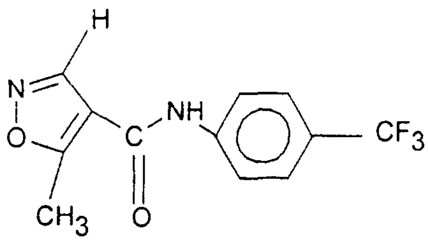

(I)

ist an sich bekannt und wird auch als N-(4-Trifluormethylphenyl)-5-methyl-isoxazole-4-carboxamid oder Leflunomid (HWA 486) bezeichnet. Die Verbindung der Formel I kann auf die im Europäischen Patent EP 0 013 376 B1 oder dem äquivalenten US-Patent US 4,284,786 beschriebene Weise erhalten werden. In den genannten Schutzrechten werden auch Verfahren zur Kristallisation aus Toluol beschrieben.

Nachteil der bekannten Verfahren zur Gewinnung der Verbindung der Formel 1 ist, daß Nebenprodukte wie N-(4-trifluormethylphenyl)-2-cyan-3-hydroxy-crotonsäureamid (im folgenden Verbindung 2) sich im wesentlichen nicht durch Kristallisation aus Lösungen enthaltend die Verbindung der Formel I und die Verbindung 2 abtrennen lassen. Die Verbindung 2 wird beispielsweise im Europäischen Patent EP 0 217 206 B1 oder dem äquivalenten US-Patent US 4,965,276 beschrieben.

Die Erfindung bezweckt durch Modifikation der Verfahrensbedingungen eine Verbindung der Formel I in hohen Ausbeuten zur Verfügung zu stellen, die im wesentlichen frei von N-(4-trif)uormethy)phenyt)-2-cyan-3-hydroxy-crotonsäureamid ist.

Die Aufgabe wird dadurch gelöst, daß man die Verbindung der Formel I, enthaltend als Nebenprodukt die Verbindung 2, in ein organisches Lösungsmittel oder in Mischungen von organischem Lösungsmittel und Wasser überführt, die Menge der Verbindung 2 in der Lösung durch eine entsprechende Analysenmethode bestimmt und eine etwa äquimolare Menge einer Base, beispielsweise $NaHCO_3$ oder $KHCO_3$ zufügt und aus der erhaltenen Lösung die Verbindung der Formel I durch Kristallisation abtrennt.

Die Erfindung betrifft daher ein Verfahren nach Anspruch 1.

Durch die Zugabe der Base verbleibt N-(4-trifluormethylphenyl)-2-cyan-3-hydroxycrotonsäureamid in der Lösung und wird bei der Kristallisation nicht zusammen mit der Verbindung der Formel I abgetrennt. Kleinere oder größere Mengen als die unter b) definierten Menge der zugesetzten Base führen zur verstärkten Nebenproduktbildung und vermindern die Ausbeute an der Verbindung der Formel I. Bei der Gewinnung der Verbindung der Formel I geht man beispielsweise so vor, daß man die Verbindung der Formel I in einem Lösungsmittel löst. Als Lösungsmittel geeignet sind beispielsweise mit Wasser mischbare Lösungsmittel wie ($C_1$-$C_4$)-Alkohole, z.B. Methanol, Ethanol, Propanol, Isopropanol, Butanol oder Isobutanol, aber auch Ketone wie Aceton oder Methylethylketon. Danach wird Wasser zugefügt. Bewährt haben sich auch Mischungen von organischen Lösungsmitteln mit Wasser, beispielsweise von etwa 40 % bis 90 % Isopropanol. Der Lösevorgang wird vorzugsweise bei erhöhter Temperatur bis zum Siedepunkt des jeweiligen Lösungsmittels durchgeführt.

In der erhaltenen Lösung wird die Menge an N-(4-trifluormethylphenyl)-2-cyan-3-hydroxy-crotonsäureamid bestimmt. Als Bestimmungsmethode geeignet sind übliche quantitative Meßmethoden wie die Hochdruckflüssigchromatographie (HPLC) oder alkalimetrische Titration. Dabei wird eine Probe aus der Lösung entnommen und in einer Standardapparatur wird die Menge an Verbindung 2 bestimmt.

Danach wird die Base der Lösung zugefügt. Gute Ergebnisse erhält man bei der Zugabe von 70 Mol % bis 130 Mol % bezogen auf die ermittelte Menge an N-(4-trifluormethylphenyl)-2-cyan-3-hydroxy-crotonsäureamid (Verbindung 2) durch HPLC. Wenn die Menge an Verbindung 2 durch alkalimetrische Titration bestimmt wird, sind Mengen von 95 Mol % bis 105 Mol % von Vorteil. Dabei wird die quantitativ ermittelte Menge der Verbindung 2 jeweils als 100 % gesetzt und die entsprechende Molmenge der jeweils zugesetzten Base wird ermittelt. Bevorzugt ist die Zugabe von 85 Mol % bis 120 Mol % , insbesondere bevorzugt von 100 Mol % bis 115 Mol %, ganz besonders bevorzugt von 108 Mol % bis 112 Mol %. Die Base kann in gelöster oder in fester Form zugefügt werden; bevorzugt ist die Zugabe in gelöster Form.

Die Reihenfolge in der die Komponenten Wasser, Lösemittel, Verbindung der Formel I und Base zur Lösung gebracht werden kann auch in einer anderen als der obengenannten Reihenfolge geschehen. Beispielsweise kann die Base auch vor Zugabe des Lösemittels zugeben werden, Wasser vor Zugabe des Lösemittels oder die Base wird

erst in die aufgeheizte Lösung gegeben.

Geeignete Basen sind beispielsweise organische Basen wie Mono-, Di- oder Trialkylamin, z.B. Trimethylamin, Triethylamin, Tripropylamin, Tributylamin, Triisobutylamin, Tripentylamin, Trihexylamin, Dibutylmethylamin, Dimethylamin oder Diethylamin, wobei die Alkylamine unsubstituiert oder ein- bis dreifach durch Phenyl oder Benzyl substituiert sind, aromatische Amine wie Anilin und substituierte Aniline, unsubstituierte und substituierte heterocyclische Amine wie Pyridin, Piperidin, Pyrrol, Indol, Pyrazin, Pyrimidin, Morpholin, Pyrazol oder Imidazol z.B. ($C_1$-$C_4$)-Alkylpyridin. Weitere geeignete Basen sind anorganische Basen wie Natriumhydrogencarbonat ($NaHCO_3$), Natriumcarbonat ($Na_2CO_3$), Kaliumhydrogencarbonat ($KHCO_3$), Kaliumcarbonat ($K_2CO_3$), Natriumhydrogenphosphat ($Na_2HPO_4$), Natriumdihydrogenphosphat ($NaH_2PO_4$), Trinatriumphosphat ($Na_3PO_4$), Kaliumhydrogenphosphat ($K_2HPO_4$), Kaliumdihydrogenphosphat ($KH_2PO_4$) oder Trikaliumphosphat ($K_3PO_4$), bevorzugt $NaHCO_3$.

Die erhaltene Lösung oder Suspension wird erwärmt und für einige Zeit auf Siedetemperatur gehalten um eine vollständige Lösung der Verbindung der Formel I sicherzustellen. Danach wird die gegebenenfalls filtrierte Lösung so langsam abgekühlt, daß sich Kristalle der Verbindung der Formel I bilden. Bevorzugt wird auf Endtemperaturen von 20 °C bis -10 °C abgekühlt, insbesondere auf Temperaturen von 10 °C bis -5 °C, ganz besonders bevorzugt auf Temperaturen von 10 °C bis 5 °C. Die Kristalle werden abgetrennt und gegebenenfalls mit Isopropanol und anschließend mit Wasser gewaschen. Das Trocknen der Substanz erfolgt bei erhöhter Temperatur bevorzugt bei 60 °C unter verminderten Druck oder bei Normaldruck. Es sind auch andere Kristallisationsmethoden möglich wie Verdampfungskristallisation oder Verdrängungskristallisation.

Ein bevorzugtes Verfahren besteht darin die Verbindung der Formel I in 80 %igem Isopropanol bei der Siedetemperatur von Isopropanol unter Normaldruck oder verminderten Druck zu lösen, die Bestimmung der Menge an N-(4-trifluormethylphenyl)-2-cyan-3-hydroxy-crotonsäureamid mit der HPLC durchzuführen, die äquimolare Menge an Base zuzufügen und anschließend die heiße Lösung langsam abzukühlen, so daß die Kristallisation bei Temperaturen von mehr als 40 °C, bevorzugt von 40 °C bis 85 °C, besonders bevorzugt von 45 °C bis 80 °C, insbesondere von 50 °C bis 70 °C, erfolgt. Die ausgefallenen Kristalle werden dann mehrmals mit Isopropanol gewaschen und unter verminderten Druck getrocknet. Die Kristallisation kann ohne Animpfen mit Kristallen der Verbindung der Formel I erfolgen oder bevorzugt in Anwesenheit von Kristallen der Verbindung der Formel I, die durch Animpfen in die Lösung eingebracht werden. Das Animpfen kann auch mehrfach bei unterschiedlichen Temperaturen erfolgen. Die Menge des Animpfmaterials hängt von der Menge der Lösung ab und kann von einem Fachmann leicht ermittelt werden.

Ein besonders bevorzugtes Verfahren zur Gewinnung der Verbindung der Formel I aus einer Lösung, enthaltend Wasser, mindestens ein organisches Lösemittel, die Verbindung der Formel I und N-(4-trifluormethylphenyl)-2-cyan-3-hydroxycrotonsäureamid, besteht darin, daß man

a) die Menge von N-(4-trifluormethylphenyl)-2-cyan-3-hydroxy-crotonsäureamid in der Lösung durch alkalimetrische Titration bestimmt,
b) die etwa äquimolare Menge an einer Base zufügt,
c) die erhaltene Mischung auf eine Temperatur von 41°C bis zur Siedetemperatur des organischen Lösungsmittels erwärmt,
d) die erhaltene Lösung mit Wasser verdünnt oder das organische Lösungsmittel abdestilliert, so daß das organische Lösungsmittel und das Wasser in einem Verhältnis von 4:1 bis 0,3:1 vorliegen und
d) die Kristallisation bei Temperaturen oberhalb von 40°C durchführt.

Bevorzugt wird die erhaltene Lösung nach Verfahrensschritt b) filtriert.

Geeignet sind die oben genannten Basen; insbesondere bevorzugt sind Natriumhydrogencarbonat, Natriumcarbonat oder Kaliumhydrogencarbonat. Gute Ergebnisse erhält man bei der Zugabe der Base von 90 Mol % bis 110 Mol % bezogen auf die ermittelte Menge von Verbindung 2, bestimmt durch alkalimetrische Titration. Dabei wird die quantitativ ermittelte Menge der Verbindung 2 jeweils als 100 % gesetzt und die entsprechende Molmenge der jeweils zugesetzten Base wird danach ermittelt. Bevorzugt ist die Zugabe von 95 Mol % bis 105 Mol %, insbesondere bevorzugt von 98 Mol % bis 102 Mol %.

Vorteilhafte Mischungen enthalten organisches Lösungsmittel und Wasser im Verhältnis von 1:1 bis 8:1, bevorzugt von 2:1 bis 6:1, insbesondere von 3:1 bis 5:1.

Die Herstellung der Lösung erfolgt vorzugsweise bei erhöhter Temperatur, insbesondere bei Temperaturen von 41°C bis zum Siedepunkt des jeweiligen Lösungsmittels. Die erwärmte Lösung wird beispielsweise für einige Zeit auf Siedetemperatur gehalten um eine vollständige Lösung der Verbindung der Formel I sicherzustellen. Der Lösevorgang kann auch bei erhöhtem Druck durchgeführt werden. Danach wird die Lösung filtriert. Der eingesetzte Filter hat einen Porendurchmesser von etwa 0,1 µm bis 200 µm. Der filtrierten Lösung wird anschließend Wasser zugegeben, das vorteilhaft die gleiche Temperatur wie die filtrierte Lösung hat, oder das organische Lösungsmittel wird abdestilliert. Die erhaltenen Lösungen enthalten vorteilhaft das organische Lösungsmittel und Wasser im Verhältnis von 4:1 bis 0,3 :1, bevorzugt von 2:1 bis 0,6:1, insbesondere bevorzugt von 1,6:1 bis 0,8:1. Danach wird langsam abgekühlt bis

zu einer minimalen Temperatur von 40°C. Die Kristalle werden abgetrennt und mit Isopropanol und anschließend mit Wasser gewaschen. Das Trocknen der Substanz erfolgt vorteilhaft bei erhöhter Temperatur bevorzugt bei 60 °C unter verminderten Druck oder bei Normaldruck.

Ein insbesondere bevorzugtes Verfahren besteht darin die Verbindung der Formel I in einer Mischung von Isopropanol und Wasser im Verhältnis von 4:1 bis 5:1 und bei Siedetemperatur von Isopropanol unter Normaldruck oder verminderten Druck zu lösen und zu filtrieren. Anschließend wird heiße Lösung mit soviel gleichwarmem Wasser versetzt, daß ein Verhältnis von Isopropanol zu Wasser von 2:1 bis 0,8:1 vorliegt. Danach erfolgt die Kristallisation bei Temperaturen von mehr als 40 °C, bevorzugt von 40 °C bis 85 °C, besonders bevorzugt von 45 °C bis 80 °C, insbesondere von 50 °C bis 70 °C. Die ausgefallenen Kristalle werden dann mehrmals mit Isopropanol gewaschen und unter verminderten Druck getrocknet.

Vorteilhaft bei der erfindungsgemäßen Gewinnung der Verbindung der Formel I ist die Reinheit von besser als 99,9 % und einem Restgehalt von N-(4-trifluormethylphenyl)-2-cyan-3-hydroxy-crotonsäureamid von weniger als 0,05 %, bestimmt durch Hochdruckflüssigchromatographie (HPLC).

Beispiel 1

Stabilität von Leflunomid gegenüber Natriumhydrogencarbonat

40 g der Verbindung der Formel I wurden im Versuch a) in 80 ml Isopropanol und 63 ml Wasser (Verhältnis Isopropanol zu Wasser wie 1,27:1) gelöst und 1 Stunde (h) bei 84 °C gerührt. Dann wurde eine Probe entnommen und über HPLC quantitativ analysiert. Anschließend wurden 0,62 g $NaHCO_3$, dies sind 5 Mol % bezogen auf die Verbindung der Formel I, zugegeben und weitere 5 h bei 84° C gerührt.
Im Versuch b) wurde ein Verhältnis von Isopropanol zu Wasser wie 4:1 eingesetzt und der Versuch analog zu a) durchgeführt, jedoch betrug die Temperatur 80 °C. Die Bildung von N-(4-trifluormethylphenyl)-2-cyan-3-hydroxy-crotonsäureamid (Verbindung 2) wurde durch HPLC-Messung über die Zeit verfolgt.
Die Ergebnisse der HPLC-Messung und die pH-Werte der Einzelmessungen sind in den Tabellen 1 und 2 zusammengefaßt. Tabelle 1 zeigt die Bildung der Verbindung 2 in Abwesenheit von Natriumhydrogencarbonat, während in der Tabelle 2 die Bildung der Verbindung 2 in Anwesenheit von Natriumhydrogencarbonat gezeigt wird.

Tabelle 1

| | Isopropanol zu Wasser wie 1,27 : 1 | | | |
|---|---|---|---|---|
| Zeit (min) | Verbindung 2 (Fl.%) | Verbindung der Formel I (Fl. %) | pH | Bemerkung |
| 0 | 0,026 | 99,88 | 4,0 | ohne $NaHCO_3$ |
| 60 | 0,082 | 99,82 | 4,0 | ohne $NaHCO_3$ |

Im Versuch a) (Tabelle 1) wurde in der Ausgangslösung ein pH-Wert von 4,0 gemessen, der Wert war auch nach 1 h unverändert. Die Menge an Verbindung 2 erhöhte sich in dieser Zeit von 0,026 Flächenprozent (Fl. %) auf 0,082 Fl. %. Im Versuch b) (Tabelle 1) erfolgte keine Bestimmung des pH-Wertes und der Menge an Verbindung 2.

Tabelle 2:

| | Isopropanol zu Wasser wie 1,27 : 1 (Versuch a)) | | | Isopropanol zu Wasser wie 4 : 1 (Versuch b)) | |
|---|---|---|---|---|---|
| Zeit (min) | Verbindung 2 (Fl. %) | Verbindung der Formel I (Fl. %) | pH | Verbindung 2 (Fl. %) | pH |
| 0 | 3,4 | 96,56 | 8,5 | n.b. | n.b. |
| 30 | 6,63 | 93,36 | 4,9 | 4,30 | n.b. |
| 60 | 6,80 | 93,17 | 4,5 | 4,51 | n.b. |
| 120 | 6,93 | 93,02 | 4,4 | 4,68 | n.b. |
| 180 | 7,10 | 92,78 | 4,3 | 5,02 | 5,7 |
| 300 | 7,22 | 92,59 | 4,3 | 5,56 | 5,7 |
| n.b. bedeutet nicht bestimmt | | | | | |

Im Versuch a) (Tabelle 2) wurde in der Lösung direkt nach Zugabe von Natriumhydrogencarbonat ein pH-Wert von 8,5 gemessen, der pH-Wert erniedrigte sich innerhalb von 5 Stunden auf 4,3. Die Bildung der Verbindung 2 nach Zugabe von Natriumhydrogencarbonat erfolgte sehr schnell. Bereits direkt nach Zugabe von Natriumhydrogencarbonat ließen sich 3,4 Fl. % der Verbindung 2 nachweisen, nach 30 Minuten (min) bereits 6,6 Fl. %. Nach 5 h wurde eine Menge von 7,2 Fl. % erreicht.
Im Versuch b) verlief die Bildung der Verbindung vergleichbar schnell. Es wurde nach 5 h eine Menge der Verbindung 2 von 5,6 Fl. % erreicht.

Die Ergebnisse zeigen deutlich, daß ein Überschuß an Natriumhydrogencarbonat im wesentlichen zur Bildung der Verbindung 2 führt. Weil der Gehalt der Verbindung 2 je nach Ansatz zur Herstellung der Verbindung I schwanken kann, ist es wichtig die benötigte Menge an Natriumhydrogencarbonat vorher durch quantitative Analyse die Menge an Verbindung 2 zu bestimmen. Dies erfolgt beispielsweise durch HPLC oder alkalimetrischer Titration.

| Quantitative HPLC-Bestimmung | | |
|---|---|---|
| Gerät | Flüssigchromatograph (Waters 2690 mit PDA-Detektor 996) | |
| Säule | Material: | Edelstahl |
| | Länge: | 125 mm |
| | Innerer Durchmesser: | 4 mm |
| Stationäre Phase | Lichrospher®100 RP 18 endcapped, Korngröße 5 µm | |
| Mobile Phase | Acetonitril | 350 Volumenteile |
| | Wasser | 650 Volumenteile |
| | Triethylamin | 5 Volumenteile |
| | Der pH-Wert wurde mit Phosphorsäure 85 % auf 4,0 eingestellt. | |
| Injektionsvolumen | 10 µl | |
| Fluß | 1,0 ml/min | |
| Detektion | UV/Vis, 210nm | |
| Laufzeit | 40 min | |
| Prüflösung | Etwa 20,0 mg der zu untersuchenden Substanz wurden in 4 ml Acetonitril gelöst und mit mobiler Phase auf 20,0 ml aufgefüllt. | |
| Berechnung | Der Gehalt an Verbindung 2 wurde berechnet, indem das arithmetische Mittel aller Injektionen gebildet wurde. | |
| | $\frac{A \cdot 100}{B}$ = Gehalt Verbindung 2 in % | |
| | A = Peakfläche Verbindung 2 im Chromatogramm der Prüflösung. | |
| | B = Summe der Peakflächen im Chromatogramm der Prüflösung | |
| Systemtest | | |

(fortgesetzt)

| Quantitative HPLC-Bestimmung | | |
| --- | --- | --- |
| Systemtest-Lösung | 20 mg 4-Trifluormethylanilin (4-TFMA) werden mit mobiler Phase auf 10,0 ml verdünnt (SS1). | |
| | 30 mg Verbindung 2 und 10 mg 3-TFMP-Isomer wurden eingewogen. 1,0 ml der Lösung SS1 und 5 ml Acetonitril wurden zugegeben. Mit mobiler Phase wurde die erhaltene Mischung auf 100,0 ml aufgefüllt (SS2) und solange geschüttelt bis die Lösung klar wurde. 100 mg der Verbindung der Formel I (Leflunomid-Referenzstandard) wurden in 2 ml Acetonitril gelöst, 1,0 ml SS2 wurde zugegeben und es wurde mit mobiler Phase auf 100,0 ml aufgefüllt. (SS3) | |
| | SS3 hatte die folgende Konzentration: 1 mg/ml Leflunomid; 0,003 mg/ml Verbindung 2; 0,0001 mg/ml 3-TFMP-Isomer; 0,0002 mg/ml 4-TFMA. | |
| Selektivität | Das Chromatogramm der Standardlösung SS3 mußte folgenden Anforderungen erfüllen: | |
| | Verbindung 2 | Relative Retentionszeit: etwa 0,13 bis 0,23 |
| | 4-TFMA | Relative Retentionszeit: etwa 0,36 bis 0,44 |
| | Leflunomid | Absolute Retentionszeit: etwa 22 bis 35 |
| Abkürzungen | 4-TFMA: 4-Trifluormethylanilin | |
| | 3-TFMP: N-(3'-Trifluormethylphenyl)-5-methylisoxazol-4-carboxamid | |

Alkalimetrische Titration

| | |
| --- | --- |
| Gerät: | Titrator mit automatischer Endpunktserkennung (z.B. Metrohm Titroprocessor 716) |
| Elektrode: | kombinierte Glaselektrode<br>(z.B. Mettler Toledo DG 112-SC) |
| Faktor der Maßlösung: | 0,05 g Bernsteinsäure wurde in 50 ml Wasser gelöst und mit 0,1 N Natriumhydroxid-Lösung titriert. |
| Durchführung: | 1,0 g der zu prüfenden Substanz wurde in 50 ml Methanol gelöst und sofort mit 0,1 N Natriumhydroxid-Lösung titriert. |

Berechnung (Faktor 0,1 N Natriumhydroxid-Lösung):

$$\frac{EW \cdot 1000}{V \cdot 59{,}05} = \text{Faktor der 0,1 N Natriumhydroxid-Lösung}$$

V = Verbrauch an 0,1 N Natriumhydroxid-Lösung
(1 ml 0,1 N Natriumhydroxid-Lösung entspricht 59,05 mg Bernsteinsäure)
EW = Einwaage Bernsteinsäure in g

Berechnung:

$$\frac{V \cdot F \cdot 27{,}02}{EW} = \text{Verbindung 2 (in \%)}$$

V = Verbrauch an 0,1 N Natriumhydroxid-Lösung in ml
F = Faktor 1 N Salzsäure
EW = Einwaage der zu prüfenden Substanz in g

Beispiel 2

Kristallisation in Anwesenheit von $NaHCO_3$

16 kg der Verbindung der Formel I (Leflunomid) wurden in 28 Liter (L) Isopropanol und Wasser gelöst, so daß die Gesamtmenge Wasser 9 L ergab. Dann wurde durch alkalimetrische Titration die Menge der Verbindung 2 in der erhaltenen Lösung ermittelt. Die äquimolare Natriumhydrogencarbonatmenge wurde berechnet und in fester Form der Lösung zugegeben (siehe Tabelle 3).
Anschließend wurde auf 78 °C bis 82 °C erhitzt, 25 min bei dieser Temperatur gerührt und dann über einen Drucktrichter in einen ebenfalls schon auf gleiche Temperatur geheizten Kessel filtriert. Der Drucktrichter wurde mit der Menge Isopropanol nachgespült, die zusammen mit eingesetztem Isopropanol ein Verhältnis von Isopropano/Wasser von 4: 1 ergab (hier etwa 4 L). Danach wurde 32 L ebenfalls auf 78 °C bis 82 °C vorgeheiztes Wasser zugesetzt (ergab ein Verhältnis von Isopropanol/Wasser von 0,8:1). Dabei trübte sich die Lösung bereits, die dann in 20 min auf etwa 65 °C gekühlt wurde, etwa 40 min bei dieser Temperatur gehalten, danach in 70 min auf etwa 40 °C gekühlt und noch 20 min nachgerührt wurde. Die kristalline Verbindung der Formel I wurde durch Zentrifugation isoliert.
Tabelle 3 faßt die Ergebnisse von drei unterschiedlichen Kristallisationen zusammen.

Tabelle 3:

| Leflunomid vor Kristallisation [kg] | Verbindung 2 vor Kristallisation [%] | $NaHCO_3$ [kg] | Ausbeute Leflunomid nach Kristallisation [kg] | Ausbeute Leflunomid nach Kristallisation [%] | Verbindung 2 nach Kristallisation (HPLC) [Fl.-%] | Reinheit Leflunomid nach Kristallisation (HPLC) [Fl.-%] |
|---|---|---|---|---|---|---|
| 17,19 | 2,44 | 0,13 | 12,7 | 79,3 | 0,03 | 99,9 |
| 17,15 | 2,20 | 0,12 | 12,8 | 80,0 | 0,02 | 99,7 |
| 16,97 | 3,22 | 0,17 | 12,6 | 78,8 | 0,02 | 99,9 |

Es wurde eine durchschnittliche Ausbeute von 79,4 % der Theorie mit nur sehr geringen Abweichungen innerhalb der einzelnen Ansätze erreicht (± 0,6 %), wobei eine Reinheit von durchschnittlich 99,93 % (HPLC Fl.-%) erreicht wurde. Die Menge der Verbindung 2 lag bei den einzelnen Ansätzen nach HPLC-Bestimmung zwischen 0,02 Fl. % und 0,04 F1. %.

Beispiel 3

Kristallisation in Anwesenheit von $NaHCO_3$

50 g der Verbindung der Formel I, enthaltend 0,46 % der Verbindung 2 - die Bestimmung der Verbindung 2 Menge erfolgte durch HPLC-Messung - wurden in 25 ml Isopropanol und 25 ml Wasser gegeben; dann wurden 28,65 mg $NaHCO_3$ zugefügt und die erhaltene Suspension wurde bei Raumtemperatur von etwa 21 °C für 15 bis 30 Minuten gerührt. Danach wurden 76,9 ml Isopropanol und 1 g Aktivkohle zugegeben, auf 80 °C erhitzt und über eine Drucknutsche filtriert. Das erhaltene Filtrat wurde auf 60 °C gekühlt, mit einigen Kristallen der Verbindung der Formel 1 angeimpft und anschließend auf 0 °C bis 5 °C gekühlt. Die erhaltenen Kristalle wurden abgesaugt, mit 37,5 ml Isopropanol und 3 mal mit 125 ml Wasser gewaschen. Ausbeute 77 % der Verbindung der Formel I, Reinheit mehr als 99,9 % nach HPLC-Messung. Der Gehalt der Verbindung 2 betrug nach HPLC Messung weniger als 0,01 % bezogen auf

die Verbindung der Formel I als 100 %.

Beispiel 4

Vergleichsbeispiel zur Kristallisation ohne Natriumhydrogencarbonat

50 g der Verbindung der Formel I, enthaltend 1,7 % der Verbindung 2 - die Bestimmung der Verbindung 2 Menge erfolgte durch HPLC-Messung - wurden in 93,75 ml Isopropanol und 25 ml Wasser suspendiert. Nach Zusatz von 1g Aktivkohle wurde die erhaltene Suspension auf 84 °C erhitzt und über eine Drucknutsche filtriert. Die filtrierte Lösung wurde mit 6,25 ml Isopropanol gewaschen. Die Lösung wurde innerhalb von 2 Stunden auf Raumtemperatur von etwa 21 °C gekühlt. Danach wurde die Lösung innerhalb von 30 Minuten auf 0 °C gekühlt und diese Temperatur wurde 2 Stunden beibehalten bis die Lösung über eine Drucknutsche abgesaugt wurde. Der erhaltene Filterkuchen wurde zweimal mit je 6,25 mf Wasser gewaschen. Die Kristalle der Verbindung der Formel 1 wurden bei 50 °C unter verminderten Druck getrocknet. Ausbeute: 41,8 g der Verbindung der Formel I, Reinheit 99,1 % nach HPLC-Messung. Der Gehalt der Verbindung 2 wurde durch HPLC-Messung auf 0,86 % bestimmt bezogen auf die Verbindung der Formel I als 100 %.

**Patentansprüche**

1. Verfahren zur Gewinnung der Verbindung der Formel 1

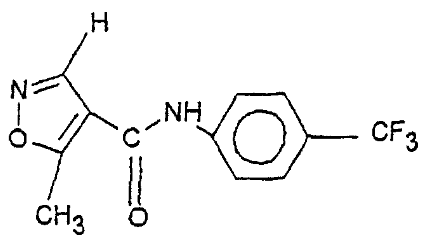

(I)

aus einer Lösung, enthaltend Wasser,
mindestens ein organisches Lösemittel aus der Gruppe der mit Wasser mischbare Lösungsmittel wie ($C_1$-$C_4$) -Alkohole, z.B. Methanol, Ethanol, Propanol, Isopropanol, Butanol oder Isobutanol, aber auch Ketone wie Aceton oder Methylethylketon, oder Mischungen der Lösemittel oder Mischungen der Lösemittel mit nicht wassermischbaren Lösungsmittel wie Ethylacetat, Toluol oder Dichlormethan,
die Verbindung der Formel I und
N-(4-trifluormethylphenyl)-2-cyan-3-hydroxy-crotonsäureamid,
das **dadurch gekennzeichnet ist, daß** man

a) die Menge von N-(4-trifluormethylphenyl)-2-cyan-3-hydroxycrotonsäureamid in der Lösung quantitativ bestimmt,
b) die Menge einer Base aus der Gruppe der anorganischen Basen wie Natriumhydrogencarbonat, Natriumcarbonat, Kaliumhydrogencarbonat, Kaliumcarbonat, Ammoniumhydrogencarbonat, Kaliumhydrogenphosphat, Natriumhydrogenphosphat, Natriumdihydrogenphosphat, Trinatriumphosphat, Kaliumdihydrogenphosphat oder Trikaliumphosphat zufügt, die 50 Mol % bis 150 Mol % der in a) bestimmten Menge von N-(4-trifluormethylphenyl)-2-cyan-3-hydroxy-crotonsäureamid entspricht,
c) die Verbindung der Formel I kristallisiert und
d) die erhaltenen Kristalle der Verbindung der Formel I von der Lösung abtrennt.

2. Verfahren gemäß Anspruch 1, **dadurch gekennzeichnet, daß** als anorganische Base Natriumhydrogencarbonat, eingesetzt wird.

3. Verfahren gemäß der Ansprüche 1 oder 2, **dadurch gekennzeichnet, daß** Isopropanol als mit Wasser mischbares Lösungsmittel eingesetzt wird.

4. Verfahren gemäß einem oder mehreren der Ansprüche 1 bis 3, **dadurch gekennzeichnet, daß** bei Temperaturen von mehr als 40 °C, bevorzugt von 41 °C bis 80 °C, insbesondere von 50 °C bis 70 °C, kristallisiert wird.

5. Verfahren gemäß einem oder mehreren der Ansprüche 1 bis 4, **dadurch gekennzeichnet, daß** die Menge der zugefügten Base von 100 Mol % bis 115 Mol %, ganz besonders bevorzugt von 108 Mol % bis 112 Mol %, der in Anspruch 1 im Verfahrensschritt a) durch quantitative Hochdruckflüssigkeitschromatographie bestimmten Menge von N-(4-trifluormethyl-phenyl)-2-cyan-3-hydroxy-crotonsäureamid entspricht.

6. Verfahren gemäß einem oder mehreren der Ansprüche 1 bis 5, **dadurch gekennzeichnet, daß** die Menge der zugefügten Base von 90 Mol % bis 110 Mol %, bevorzugt von 95 Mol % bis 105 Mol %, insbesondere bevorzugt von 98 Mol % bis 102 Mol %, der in Anspruch 1 im Verfahrensschritt a) durch alkalimetrische Titration bestimmten Menge von N-(4-trifluormethyl-phenyl)-2-cyan-3-hydroxy-crotonsäureamid entspricht.

7. Verfahren gemäß einem oder mehreren der Ansprüche 1 bis 6, **dadurch gekennzeichnet, daß** die Kristallisation in Anwesenheit von zugefügten Kristallen der Verbindung der Formel I durchgeführt wird.

8. Verfahren gemäß einem oder mehreren der Ansprüche 1 bis 7, **dadurch gekennzeichnet, daß** die erhaltene Lösung nach Verfahrensschritt b) gemäß Anspruch 1 filtriert wird.

9. Verfahren gemäß einem oder mehreren der Ansprüche 1 bis 8, **dadurch gekennzeichnet, daß** die Mischung aus organischen Lösungsmittel und Wasser gemäß Verfahrensschritt b) auf eine Temperatur von 40 °C bis 85 °C erwärmt wird.

10. Verfahren gemäß einem oder mehreren der Ansprüche 1 bis 9, **dadurch gekennzeichnet, daß** das Verhältnis von organischen Lösungsmittel zu Wasser bei Verfahrensschritt a) von 1 : 1 bis 8 : 1, bevorzugt von 2 : 1 bis 6 : 1, insbesondere von 3 : 1 bis 5 : 1, beträgt.

11. Verfahren gemäß einem oder mehreren der Ansprüche 1 bis 10, **dadurch gekennzeichnet, daß** das Verhältnis von organischen Lösungsmittel zu Wasser im Verfahrensschritt c) von 4 : 1 bis 0,3 : 1, insbesondere von 2 : 1 bis 0,6 : 1, bevorzugt von 1,6 : 1 bis 0,8 : 1, beträgt.

## Claims

1. A process for obtaining the compound of the formula 1

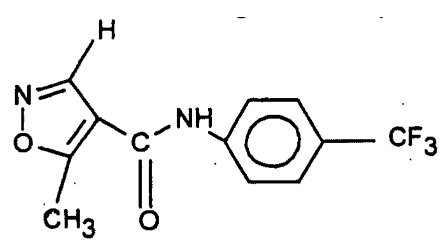

(I)

from a solution containing water, at least one organic solvent selected from the group comprising water-miscible solvents, such as ($C_1$-$C_4$)-alcohols, e.g. methanol, ethanol, propanol, isopropanol, butanol or isobutanol, but also ketones, such as acetone or methyl ethyl ketone, or mixtures of the solvents or mixtures of the solvents with water-immiscible solvents, such as ethyl acetate, toluene or dichloromethane, the compound of the formula I and N-(4-trifluoromethylphenyl)-2-cyano-3-hydroxycrotonamide, which comprises

a) quantitatively determining the amount of N-(4-trifluoromethylphenyl)-2-cyano-3-hydroxycrotonamide in the solution,
b) adding the amount of a base selected from the group comprising inorganic bases, such as sodium bicarbonate, sodium carbonate, potassium bicarbonate, potassium carbonate, ammonium bicarbonate, potassium hydrogen phosphate, sodium hydrogen phosphate, sodium dihydrogen phosphate, trisodium phosphate, potassium dihydrogen phosphate or tripotassium phosphate,which corresponds to from 50 mol% to 150 mol% of the amount of N-(4- trifluoromethylphenyl)-2-cyano-3-hydroxycrotonamide determined in a),
c) crystallizing the compound of the formula I and
d) separating the resulting crystals of the compound of the formula I from the solution.

2. The process as claimed in claim 1, wherein the inorganic base used is sodium bicarbonate.

3. The process as claimed in claim 1 or 2, wherein the water-miscible solvent used is isopropanol.

4. The process as claimed in one or more of claims 1 to 3, wherein crystallization takes place at temperatures of more than 40°C, preferably of 41°C to 80°C, in particular of 50°C to 70°C.

5. The process as claimed in one or more of claims 1 to 4, wherein the amount of added base corresponds to from 100 mol% to 115 mol%; very particularly preferably from 108 mol% to 112 mol%, of the amount of N-(4-trifluoromethylphenyl)-2-cyano-3-hydroxy-crotonamide determined by quantitative high pressure liquid chromatography in process step a) in claim 1.

6. The process as claimed in one or more of claims 1 to 5, wherein the amount of added base corresponds to from 90 mol% to 110 mol%, preferably from 95 mol% to 105 mol%, particularly preferably from 98 mol% to 102 mol%, of the amount of N-(4-trifluoromethylphenyl)-2-cyano-3-hydroxycrotonamide determined by alkalimetric titration in process step a) in claim 1.

7. The process as claimed in one or more of claims 1 to 6, wherein the crystallization is carried out in the presence of added crystals of the compound of the formula 1.

8. The process as claimed in one or more of claims 1 to 7, wherein the solution obtained is filtered after process step b) according to claim 1.

9. The process as claimed in one or more of claims 1 to 8, wherein the mixture of organic solvent in water according to process step b) is heated to a temperature of 40°C to 85°C.

10. The process as claimed in one or more of claims 1 to 9, wherein the ratio of organic solvent to water in process step a) is from 1 : 1 to 8 : 1, preferably from 2 : 1 to 6 : 1, in particular from 3 : 1 to 5 : 1.

11. The process as claimed in one or more of claims 1 to 10, wherein the ratio of organic solvent to water in process step c) is from 4 : 1 to 0.3 : 1, in particular from 2 : 1 to 0.6 : 1, preferably from 1.6 : 1 to 0.8 : 1.

## Revendications

1. Procédé pour l'obtention du composé de formule I

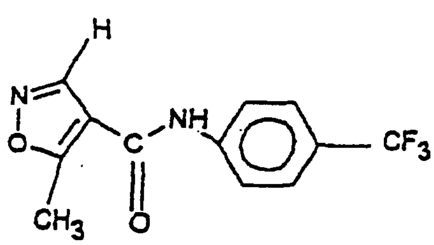

(I)

à partir d'une solution contenant de l'eau, au moins un solvant organique pris dans le groupe des solvants miscibles à l'eau, tels que des alcools en $C_1$-$C_4$, par exemple le méthanol, l'éthanol, le propanol, l'isopropanol, le butanol ou l'isobutanol, ainsi que des cétones telles que l'acétone ou la méthyléthylcétone, ou des mélanges de solvants ou mélanges de solvants avec des solvants non miscibles à l'eau, tels que l'acétate d'éthyle, le toluène ou le dichlorométhane,
le composé de formule I et
le N-(4-trifluorométhylphényl)-2-cyano-3-hydroxycrotonamide, **caractérisé en ce que**

a) on détermine quantitativement la quantité de N-(4-trifluorométhylphényl)-2-cyano-3-hydroxycrotonamide dans la solution,
b) on ajoute la quantité d'une base prise dans le groupe des bases inorganiques, comme le bicarbonate de

sodium, le carbonate de sodium, le bicarbonate de potassium, le carbonate de potassium, le bicarbonate d'ammonium, l'hydrogénophosphate de potassium, l'hydrogénophosphate de sodium, le dihydrogénophosphate de sodium, le phosphate trisodique, le dihydrogénophosphate de potassium ou le phosphate tripotassique, qui correspond à la quantité de 50 % molaires-150 % molaires de la quantité de N-(4-trifluorométhylphényl)-2-cyano-3-hydroxycrotonamide déterminée au point a),

c) on cristallise le composé de formule 1 et

d) on isole à partir de la solution les cristaux du composé de formule I obtenus.

2. Procédé selon la revendication 1, **caractérisé en ce qu'**on utilise comme base inorganique le bicarbonate de sodium.

3. Procédé selon les revendications 1 ou 2, **caractérisé en ce qu'**on utilise l'isopropanol en tant que solvant miscible à l'eau.

4. Procédé selon une ou plusieurs des revendications 1 à 3, **caractérisé en ce qu'**on réalise la cristallisation à une température supérieure à 40°C, de préférence dans un domaine de 41°C à 80°C, en particulier de 50°C à 70°C.

5. Procédé selon une ou plusieurs des revendications 1 à 4, **caractérisé en ce que** la quantité de la base ajoutée correspond à une quantité de 100 % molaires à 115 % molaires, de manière particulièrement préférée de 108 % molaires à 112 % molaires, de la quantité de N-(4-trifluorométhylphényl)-2-cyano-3-hydroxy-crotonamide déterminée à la revendication 1 étape a), par chromatographie liquide haute pression quantitative.

6. Procédé selon une ou plusieurs des revendications 1 à 5, **caractérisé en ce que** la quantité de la base ajoutée est de 90 % molaires à 110 % molaires, de préférence de 95 % molaires à 105 % molaires, de manière particulièrement préférée de 98 % molaires à 102 % molaires de la quantité de N-(4-trifluorométhylphényl)-2-cyano-3-hydroxy-crotonamide déterminée par titrage alkalimétrique à l'étape a) de la revendication 1.

7. Procédé selon une ou plusieurs des revendications 1 à 6, **caractérisé en ce qu'**on met en oeuvre la cristallisation en présence des cristaux ajoutés du composé de formule I.

8. Procédé selon une ou plusieurs des revendications 1 à 7, **caractérisé en ce qu'**on filtre la solution obtenue selon l'étape b) de la revendication 1.

9. Procédé selon une ou plusieurs des revendications 1 à 8, **caractérisé en ce qu'**on chauffe le mélange de solvant organique et d'eau obtenu selon l'étape réactionnelle b) à une température de 40°C à 85°C.

10. Procédé selon une ou plusieurs des revendications 1 à 9, **caractérisé en ce que** le rapport du solvant organique à l'eau à l'étape a) est de 1:1 à 8:1, de préférence de 2:1 à 6:1, en particulier de 3:1 à 5:1.

11. Procédé selon une ou plusieurs des revendications 1 à 10, **caractérisé en ce que** le rapport des solvants organiques à l'eau dans l'étape réactionnelle c) est de 4:1 à 0,3:1, en particulier de 2:1 à 0,6:1, de préférence de 1,6:1 à 0,8:1.